# EUROPÄISCHE PATENTSCHRIFT

(11) **EP 0 672 700 B1**
(45) Veröffentlichungstag und Bekanntmachung des Hinweises auf die Patenterteilung: **02.06.1999**
(21) Anmeldenummer: 95103318.2
(22) Anmeldetag: 08.03.1995
(51) Int. Cl.: C08G 69/06, C07D 239/54, C07D 239/46, C07D 473/18, C07D 473/34, C08G 69/10

(54) **PNA-Synthese unter Verwendung einer gegen schwache Säuren labilen Amino-Schutzgruppe**
PNA-synthesis using an aminoprotecting group which is labile against weak acids
Synthèse de PNA en utilisant un groupe aminoprotecteur labile contre des acides faibles

(30) Priorität: 14.03.1994 DE 4408531
(43) Veröffentlichungstag der Anmeldung: 20.09.1995
(73) Patentinhaber: HOECHST AKTIENGESELLSCHAFT, 65929 Frankfurt am Main (DE)
(72) Erfinder: Breipohl, Gerhard, Dr., D-60529 Frankfurt (DE); Uhlmann, Eugen, Dr., D-61479 Glashütten (DE)

(56) Entgegenhaltungen:
- WO-A-92/20702
- WO-A-93/12129
- ANGEWANDTE CHEMIE, Bd.31, Nr.8, 8. August 1992 Seiten 1008 - 1010 CHRIS MEIER ET AL
- JOURNAL OF THE AMERICAN CHEMICAL SOCIETY, Bd.114, Nr.5, 26. Februar 1992 Seiten 1895 - 1897 MICHAEL EGHOLM ETAL
- JOURNAL OF THE AMERICAN CHEMICAL SOCIETY, Bd.114, Nr.24, 18. November 1992 Seiten 9677 - 9678 MICHAEL EGHOLM ET AL
- THE JOURNAL OF ORGANIC CHEMISTRY, Bd.59, Nr.19, 23. September 1994 Seiten 5767 - 5773 KIM L. DUEHOLM ET AL

## Beschreibung

Peptide bzw. Polyamide Nucleic Acids (PNA) sind DNA-analoge Verbindungen in denen das Deoxyribose-Phosphat-Gerüst durch ein Peptid-Oligomer ersetzt wurde. Die bisher in der Literatur (Michael Egholm, Peter E. Nielsen, Ole Buchardt and Rolf H. Berg, J. Am. Chem. Soc. 1992, 114, 9677-9678; Ole Buchardt, Michael Egholm, Peter E. Nielsen and Rolf H. Berg, WO 92/20702) beschriebenen Synthesen verwenden zum temporären Schutz der Aminogruppe des Monomers die säurelabile tert.-Butyloxycarbonyl (Boc)-Schutzgruppe, die durch mittelstarke Säuren, wie z.B. Trifluoressigsäure abgespalten wird. Die Festphasensynthese von Oligomeren folgt dabei dem üblichen Peptidsyntheseverfahren, wie es z.B. von Merrifield (B. Merrifield, J. Am. Chem. Soc., 1963, 85, 2149) beschrieben wurde. Die Abspaltung des PNA-Oligomeren vom festen Träger erfolgt dabei mit einer starken Säure, üblicherweise mit flüssigem Fluorwasserstoff. Die wiederholte Behandlung mit Trifluoressigsäure und die anschließende Spaltung mit Fluorwasserstoff ist mit der Synthese von gemischten PNA/DNA-Sequenzen nicht kompatibel, da unter diesen Bedingungen die nucleosidische Bindung nicht stabil ist. Insbesondere werden die Purinnucleotide Desoxyguanosin und Desoxyadenosin mit starken Säuren an der N-glykosidischen Bindung rasch gespalten. Weiterhin wäre für die Synthese von derartigen Molekülen eine Verwendung von den üblichen DNA-Synthesizern und einer weitgehenden Beibehaltung der in diesen Geräten verwendeten Chemie besonders wünschenswert.

Ziel der Erfindung ist, ein Syntheseverfahren mit einer gegen schwache Säuren labilen temporären Amino-Schutzgruppe für den Aufbau der PNA-Oligomeren zu entwickeln, das eine Abspaltung vom festen Träger unter den üblicherweise für Oligonucleotide verwendeten basischen Verfahren erlaubt.

Die nachfolgende Erfindung beschreibt ein Verfahren zur Herstellung von PNA-Oligomeren der Formel I 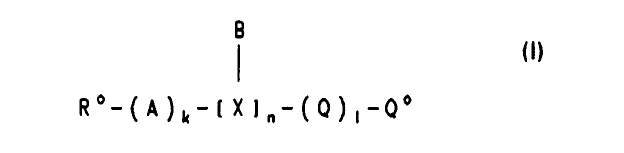 worin
B/X für 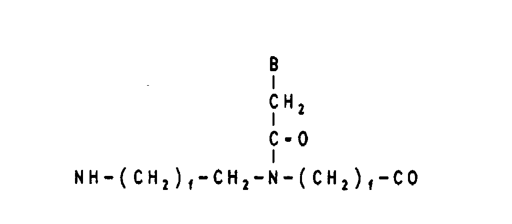 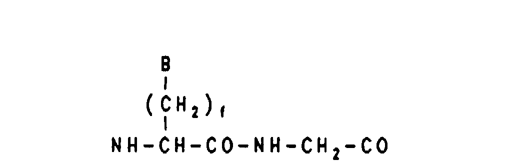 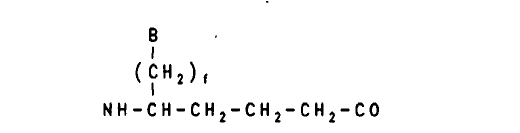 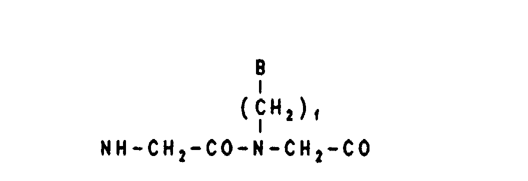 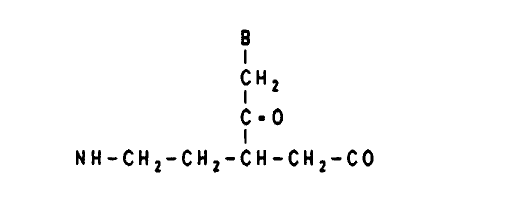 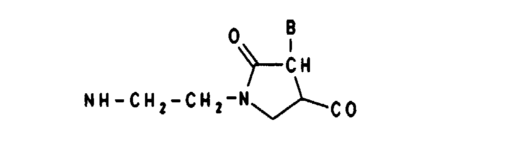 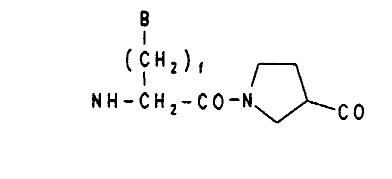 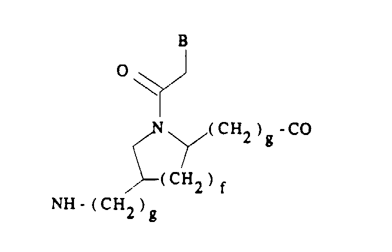 bevorzugt 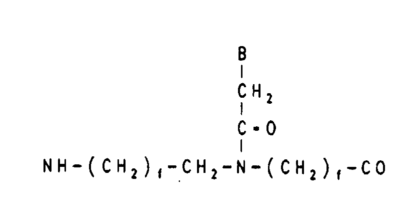 mit f = 1 - 4, bevorzugt 1 oder 2 und g = Null - 3, bevorzugt Null - 2, steht;
- R⁰: Wasserstoff, C₁-C₁₈-Alkanoyl, C₁-C₁₈-Alkoxycarbonyl, C₃-C₈-Cycloalkanoyl, C₇-C₁₅-Aroyl, C₃-C₁₃-Heteroaroyl, oder eine Gruppe bedeutet, die die intrazelluläre Aufnahme des Oligomers begünstigt oder die bei der Hybridisierung mit der target-Nucleinsäure interagiert;
- A: ein Aminosäurerest, bevorzugt aus der Reihe Glycin, Leucin, Histidin, Phenylalanin, Cystein, Lysin, Arginin, Asparaginsäure, Glutaminsäure, Prolin, Tetrahydroisochinolin-3-carbonsäure, Octahydroindol-2-carbonsäure und N-(2-Aminoethyl)glycin bedeutet;
- k: eine ganze Zahl von Null bis 10, bevorzugt Null bis 6 ist;
- Q: ein Aminosäurerest, bevorzugt aus der Reihe Glycin, Leucin, Histidin, Phenylalanin, Cystein, Lysin, Arginin, Asparaginsäure, Glutaminsäure, Prolin, Tetrahydroisochinolin-3-carbonsäure, Octahydroindol-2-carbonsäure und N-(2-Aminoethyl)glycin bedeutet;
- I: eine ganze Zahl von Null bis 10, bevorzugt Null bis 6 ist;
- B: für eine in der Nucleotidchemie übliche Nucleobase, beispielsweise für natürliche Nucleobasen ausgewählt aus Adenin, Cytosin, Guanin, Thymin und Uracil oder nichtnatürliche Nucleobasen ausgewählt aus Purin, 2.6-Diaminopurin, 7-Deazaadenin, 7-Deazaguanin, N⁴N⁴-Ethanocytosin, N⁶N⁶-Ethano-2.6-diaminopurin, 5-Methylcytosin, 5-(C₃-C₆)-Alkinyl-uracil, 5-(C₃-C₆)-Alkinyl-cytosin, 5-Fluor-uracil oder Pseudoisocytosin, 2-Hydroxy-5-methyl-4-triazolopyrimidin oder deren Prodrugformen, oder auch für Basenersatzverbindungen, wie z.B. Imidazol, Nitro-lmidazol und Triazol, steht;
- Q^{o}: Hydroxy, NH₂ oder NHR" bedeutet mit R" = C₁-C₁₈-Alkyl, C₂-C₁₈-Aminoalkyl oder C₂-C₁₈-Hydroxyalkyl; und
- n: eine ganze Zahl von 1 - 50, bevorzugt 4 - 35 ist,
das dadurch gekennzeichnet ist, daß man
a) auf einen polymeren Träger der Formel II

   L-[Polymer] (II),

   der mit einer Ankergruppe L versehen ist, die latent den Rest Q° enthält entweder zuerst mit einem für die Festphasensynthese üblichen Verfahren Aminosäuren (Q') aufkuppelt,
b) die gegen schwache Säuren labile Schutzgruppe PG mittels eines geeigneten Reagenzes abspaltet,
c) die Schritte a und b (I-1)fach wiederholt,
d) auf die dabei als Zwischenprodukt entstehende Verbindung der Formel III

   (Q')_{I}-L-[Polymer] (III),

   worin L wie oben definiert ist, Q' für eine Aminosäure Q steht, bei der die gegebenenfalls vorhandenen Seitenkettenfunktionen geschützt sind, und I für eine ganze Zahl von Null bis 10 steht oder direkt auf den polymeren Träger der Formel II eine Verbindung der Formel IV 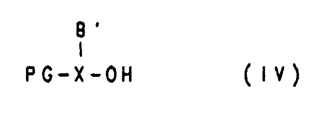 worin
   PG für eine gegen schwache Säuren labile Aminoschutzgruppe und
   B'/X für einen Baustein wie in Formel I definiert steht, mit einer an der exocyclischen Amino- oder Hydroxyfunktion gegebenenfalls geschützten Nucleobase, worin
   B' für in der Nucleotidchemie übliche Nucleobasen, beispielsweise für natürliche Nucleobasen, ausgewählt aus Adenin, Cytosin, Guanin, Thymin und Uracil oder nichtnatürliche Nucleobasen, ausgewählt aus Purin, 2.6-Diaminopurin, 7-Deazaadenin, 7-Deazaguanin, N⁴N⁴-Ethanocytosin, N⁶N⁶-Ethano-2.6-diaminopurin, 5-Methylcytosin, 5-(C₃-C₆)-Alkinyl-uracil, 5-(C₃-C₆)-Alkinyl-cytosin, 5-Fluoruracil oder Pseudoisocytosin 2-Hydroxy-5-methyl-4-triazolopyrimidin oder deren Prodrugformen, deren exocyclische Amino bzw. Hydroxygruppen gegebenenfalls durch geeignete bekannte Schutzgruppen, wie die Benzoyl-, Isobutanoyl-, Acetyl-, Phenoxyacetyl-, 4-(t-Butyl)-benzoyl-, 4-(t-Butyl)-Phenoxyacetyl-, 4-(Methoxy)-benzoyl-, 2-(4-Nitrophenyl)ethyl-oxycarbonyl-, 2-(2,4-Dinitrophenyl)ethyl-oxycarbonyl-, 9-Fluorenylmethoxycarbonyl-, Diphenylcarbamoyl oder Formamidin-Gruppe, bevorzugt die Benzoyl, Isobutanoyl, 4-(t-Butyl)benzoyl-, 2-(4-Nitrophenyl)ethyl-oxycarbonyl-, 2-(2,4-Dinitrophenyl)ethyl-oxycarbonyl-, 9-Fluorenylmethoxycarbonyl-, und für Guanin durch eine Kombination von 2-N-Acetyl mit der 6-O-Diphenylcarbamoyl-Gruppe, geschützt sind, oder auch für Basenersatzverbindungen, wie z.B. Imidazol, Nitro-lmidazol und Triazol, stehen, unter Verwendung der in der Peptidchemie üblichen Kupplungsreagenzien aufkuppelt,
e) die temporäre gegen schwache Säuren labile Schutzgruppe PG mittels eines geeigneten Reagenzes abspaltet,
f) die Schritte d und e (n-1)fach wiederholt,
g) mit einem für die Festphasensynthese üblichen Verfahren weitere Aminosäuren (A') aufkuppelt,
h) die gegen schwache Säuren labile Schutzgruppe PG mittels eines geeigneten Reagenzes abspaltet,
i) die Schritte g und h (k-1)fach wiederholt,
j) für den Fall, daß R⁰ nicht Wasserstoff ist, den Rest R⁰ mit einem üblichen Verfahren einführt,
k) aus der als Zwischenverbindung erhaltenen Verbindung der Formel la 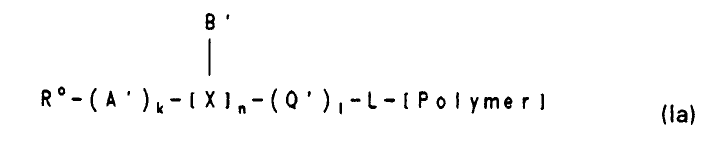 worin R⁰, k, B'/X, n, Q', und I wie oben definiert sind, A' für eine Aminosäure A steht, die gegebenenfalls in der Seitenkette geschützt ist,
   und L für eine Ankergruppe steht,
   die Verbindung der Formel I mittels eines Abspaltungsreagenzes vom polymeren Träger abspaltet wobei gleichzeitig oder auch anschließend die an der exocyclischen Amino- bzw. Hydroxyfunktion der Nucleobasen und an den Seitenketten der Aminosäuren gegebenenfalls vorhandenen Schutzgruppen abgespalten werden.

Nachfolgendes Syntheseschema für PNA zeigt diesen Reaktionsablauf:

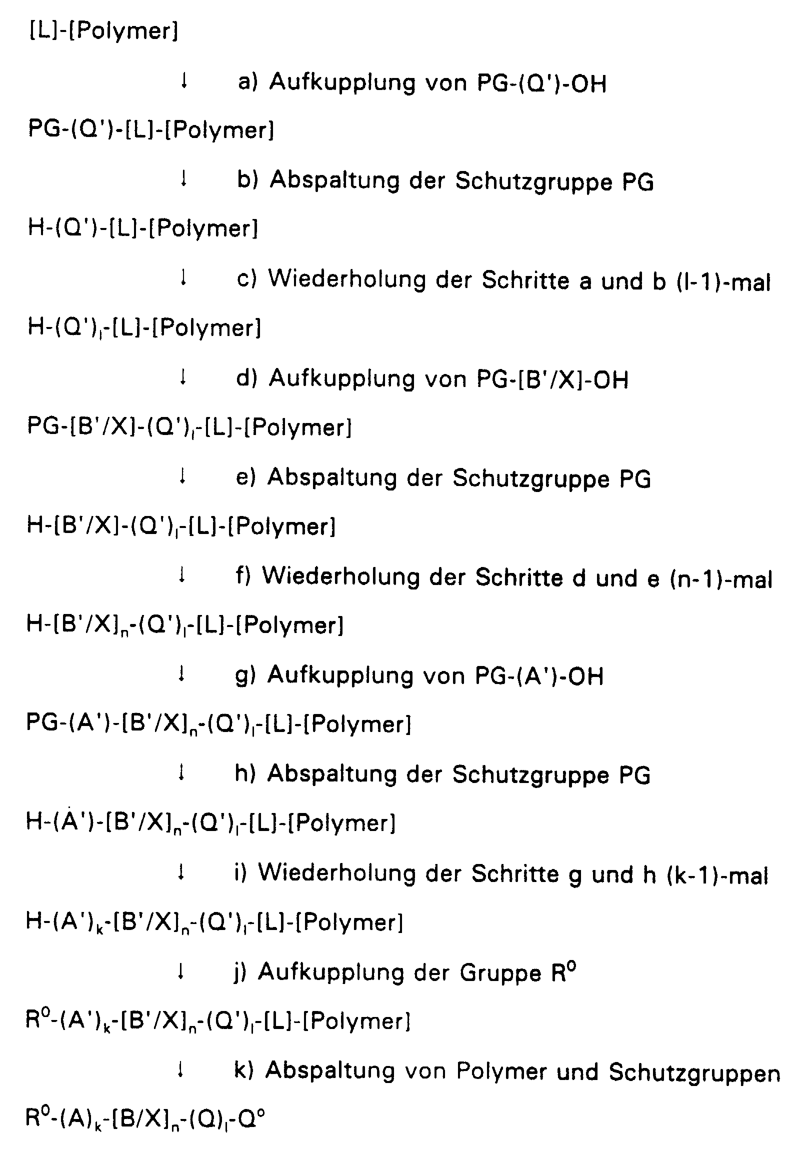

Gruppen, die die intrazelluläre Aufnahme des Oligomers begünstigen sind zum Beispiel Alkanoyl- und Alkoxycarbonylverbindungen mit verschiedenen lipophilen Resten wie -(CH₂)ₓ-CH₃, worin x eine ganze Zahl von 6-18 bedeutet, -(CH₂)ₙ-CH =CH-(CH₂)ₘ-CH₃, worin n und m unabhängig voneinander eine ganze Zahl von 6 bis 12 bedeuten, -(CH₂CH₂O)₄-(CH₂)₉-CH₃, -(CH₂CH₂O)₈ - (CH₂)₁₃-CH₃ und -(CH₂CH₂O)₇-(CH₂)₁₅-CH₃, aber auch Steroid-Reste wie Cholesteryl oder Vitamin-Reste wie Vitamin E, Vitamin A oder Vitamin D und andere Konjugate, die natürliche Carriersysteme ausnutzen wie Gallensäure, Folsäure, 2-(N-Alkyl, N-Alkoxy)-Aminoanthrachinon und Konjugate der Mannose und Peptide der entsprechenden Rezeptoren, die zur rezeptorvermittelten Endozytose der Oligomeren führen wie EGF (Epidermal Growth Factor), Bradykinin und PDGF (Platelet Derived Growth Factor). Unter Markierungs-Gruppen sind fluoreszierende Gruppen beispielsweise von Dansyl- (N-Dimethyl-1-aminonaphthyl-5-sulfonyl-), Fluorescein- oder Coumarin-Derivaten oder chemilumineszierende Gruppen beispielsweise von Acridin-Derivaten zu verstehen sowie das über ELISA nachweisbare Digoxygenin-System, die über das Biotin/Avidin-System nachweisbare Biotin-Gruppe oder aber Linker-Arme mit funktionellen Gruppen, die eine nachträgliche Derivatisierung mit nachweisbaren Reporter-Gruppen gestatten, beispielsweise ein Aminoalkyl-Linker, der mit einem Acridinium-Aktivester zur Chemilumineszenz-Probe umgesetzt wird. Typische Markierungsgruppen sind:

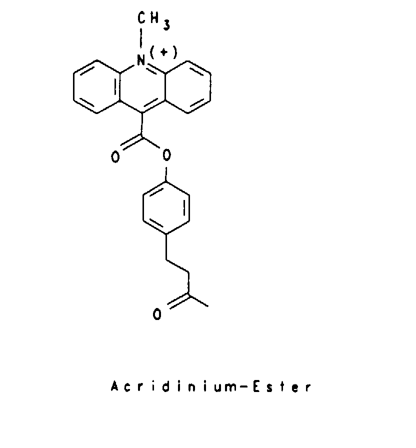

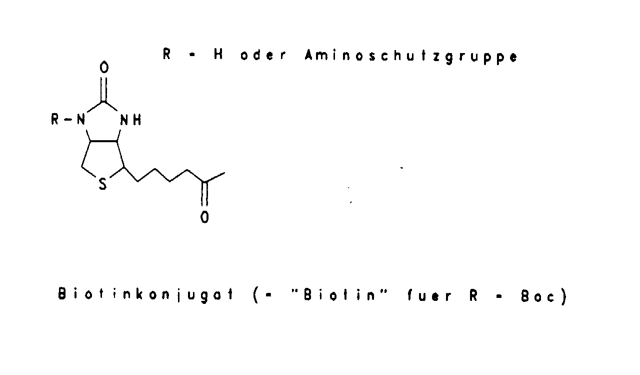

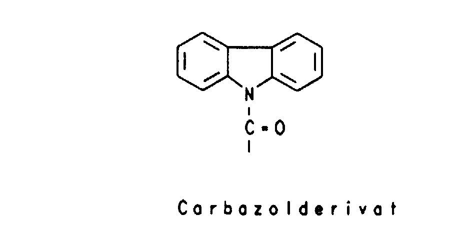

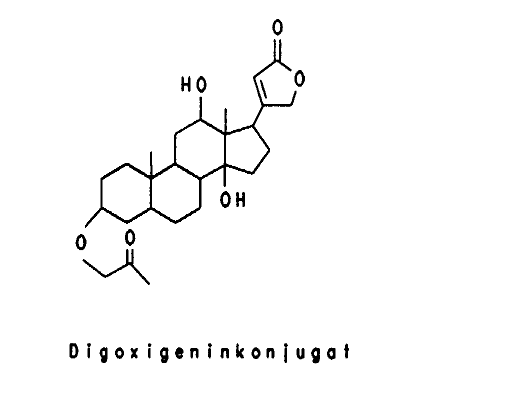

Gruppen, die bei der Hybridisierung des Oligomers an die target-Nucleinsäure diese unter Bindung, Quervernetzung oder Spaltung angreifen sind zum Beispiel Acridin-, Psoralen-, Phenanthridin, Naphtochinon-, Daunomycin- oder Chlorethylaminoaryl-Konjugate. Typische interkalierende und quervernetzende Reste sind: 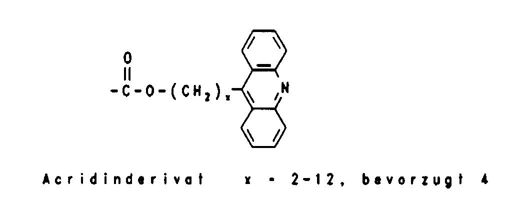 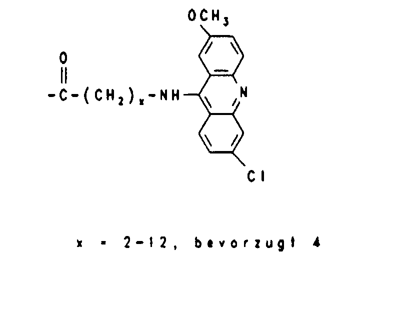 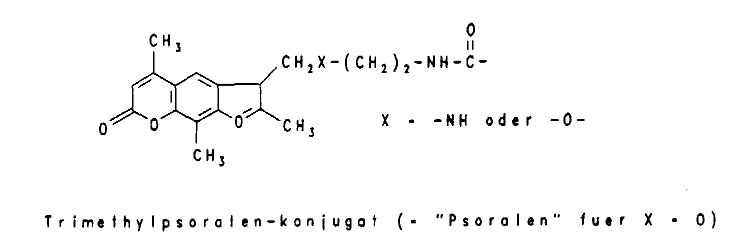 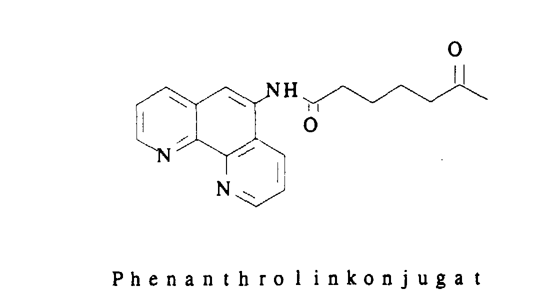 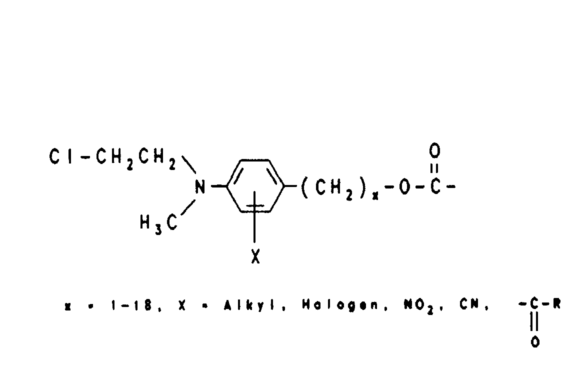 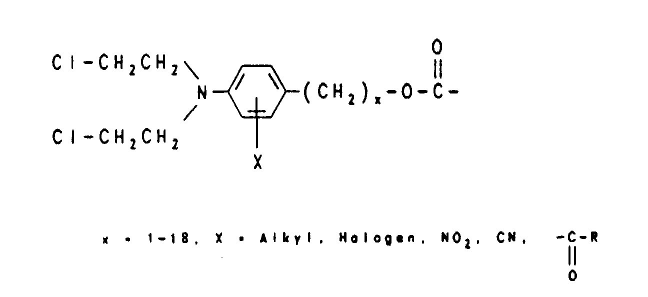 Ankergruppen L, die latent die Funktion Q^{o} enthalten sind zum Beispiel bei George Barany, Nancy Kneib-Cordonier and Daniel G. Mullen., Int. J. Peptide Protein Res., 1987, 30, 705-739; beschrieben.

Polymere Träger, die mit einer Ankergruppe versehen sind, die latent die Gruppe Q° enthalten, sind beispielsweise p-Nitrobenzophenonoxim-Polystyrol-Harz (E.T. Kaiser, S.H. Nakagawa, J. Org. Chem. 1983, 48, 678-685), 4-(2-Hydroxyethylsulfonyl)benzoyl-Harz oder die mit einer primären Aminogruppe funktionalisierten polymeren Träger, wie z.B. ®polyHIPE, ®Tentagel, ®Controlled Pore Glass, Polystyrol, auf die eine der latent die Gruppe Q^{o} enthaltende Ankergruppen wie z.B. 4-Hydroxymethylbenzoesäure (E.Atherton, C.J. Logan, R.C. Sheppard, J.Chem.Soc., Perkin Trans. I, 538-546 (1981)), 9-Hydroxymethylfluoren-4-carbonsäure (M. Mutter, D. Bellof, Helv. Chim. Acta 67, 2009-2016 (1984)), 4-(2-Hydroxyethylsulfonyl)benzoesäure (R. Schwyzer, E. Felder, P. Failli, Helv. Chim. Acta 67, 1316-1327 (1984)); (9-(Hydroxymethyl)-2-fluorenyl-essigsäure (Y.Z. Liu,S.H. Ding, J.Y. Chu, A.M. Felix, Int. J. Peptide Protein Res. 35, 95-98(1990)), N-[9-(Hydroxymethyl)-2-fluorenyl]-bernsteinsäuremonoamid; 4-(2-Hydroxyethyl)-3-nitrobenzoesäure (F. Albericio, E. Giralt, R. Eritja, Tetrahedron Lett. 1991, 1515-1518), Bernsteinsäure-mono(amino-C₂-C₁₆)alkyl)ester, Oxalsäure-mono(amino-C₂-C₁₆)alkyl)ester und ähnliche aufgekuppelt sind.

Bevorzugt verwendet werden die folgenden Ankergruppen oder schon an den polymeren Träger gebundenen Ankergruppen:
p-Nitrobenzophenonoxim-Polystyrol-Harz, 4-(2-Hydroxyethylsulfonyl)benzoyl-Harz oder die auf mit einer primären Aminogruppe funktionalisierten Träger vom Typ Tentagel, Controlled Pore Glass, Polystyrol, latent die Gruppe Q^{o} enthaltenden gekuppelten Ankergruppen, wie 4-Hydroxymethylbenzoesäure, 4-(2-Hydroxyethylsulfonyl)benzoesäure, N-[9-(Hydroxymethyl)-2-fluorenyl]-bernsteinsäuremonoamid, Bernsteinsäure-mono(amino-C₂-C₁₆)alkyl)ester oder Oxalsäure-mono(amino-C₂-C₁₆)alkyl)ester.

Gegen schwache Säuren labile Schutzgruppen PG sind z.B. 1-(1-Adamantyl)1-methylethoxycarbonyl (Adpoc), 1-(3,5.-di-tert.-butylphenyl)1-methylethoxycarbonyl (t-Bumeoc), 1-Methyl1-(4-biphenyl)ethyloxycarbonyl (Bpoc), 3.5-Dimethoxyphenyl-2-propyl-2-oxycarbonyl (Ddz) oder vom Trityl-Typ, wie Triphenylmethyl (Trt), (4-Methoxyphenyl)diphenylmethyl (Mmt), (4-Methylphenyl)diphenylmethyl (Mtt), Di-(4-methoxyphenyl)phenylmethyl (Dmt), 9-(9-Phenyl)xanthenyl (Pixyl), besonders bevorzugt werden Schutzgruppen vom Trityl-Typ, wie Trt, Mmt, Dmt verwendet, ganz besonders bevorzugt wird die Mmt-Schutzgruppe verwendet.

Die in Schritt a des obigen Syntheseverfahrens benutzten in der Peptidsynthese üblichen Aktivierungsmethoden sind z. B. in Houben-Weyl, Methoden der organischen Chemie, Band 15/2, Georg Thieme Verlag Stuttgart 1974, beschrieben. Weitere Reagenzien wie z.B. BOP (B. Castro, J.R. Dormoy, G. Evin and C. Selve, Tetrahedron Lett. 1975, 1219-1222), PyBOP (J. Coste, D. Le-Nguyen und B. Castro, Tetrahedron Lett. 1990, 205-208), BroP (J. Coste, M.-N. Dufour, A. Pantaloni und B. Castro, Tetrahedron Lett. 1990, 669-672), PyBroP (J. Coste, E. Frerot, P. Jouin und B. Castro, Tetrahedron Lett. 1991, 1967-1970) und Uronium-Reagenzien, wie z.B. HBTU (V. Dourtoglou, B. Gross, V. Lambropoulou, C. Zioudrou, Synthesis 1984, 572-574), TBTU, TPTU, TSTU, TNTU (R. Knorr, A. Trzeciak, W. Bannwarth and D. Gillessen, Tetrahedron Letters 1989, 1927-1930), TOTU (EP-A-0 460 446), HATU (L.A. Carpino, J. Am. Chem. Soc. 1993, 115, 4397-4398), HAPyU, TAPipU (A. Ehrlich, S. Rothemund, M. Brudel, M. Beyermann, L.A. Carpino und M. Bienert, Tetrahedron Lett. 1993, 4781-4784), BOI (K. Akaji, N. Kuriyama, T. Kimura, Y. Fujiwara und Y. Kiso, Tetrahedron Lett. 1992, 3177-3180) oder 2,4,6-Mesitylensulfonyl-3-nitro-1,2,4-triazolid (MSNT) (B. Blankemeyer-Menge, M. Nimitz und R. Frank, Tetrahedron Lett. 1990, 1701-1704), 2,5-Diphenyl-2,3-dihydro-3-oxo-4-hydroxythiophendioxid (TDO) (R. Kirstgen, R.C. Sheppard, W. Steglich, J. Chem. Soc. Chem. Commun. 1987, 1870-1871) oder aktivierte Ester (D. Hudson) Peptide Res. 1990, 51-55) sind in den jeweiligen Literaturstellen beschrieben.

Bevorzugt ist die Verwendung von Carbodiimiden, z.B. Dicyclohexylcarbodiimid oder Diisopropylcarbodiimid. Bevorzugt verwendet werden ebenfalls Phosphonium-Reagenzien, wie z.B. PyBOP oder PyBroP, Uronium-Reagenzien, wie z.B. HBTU, TBTU, TPTU, TSTU, TNTU, TOTU, HATU oder BOI.

Die Kupplung kann dabei direkt durch Addition von Aminosäurederivat oder PNA-Monomer der Formel IV mit dem Aktivierungsreagenz und gegebenenfalls unter Zusatz von Additiven wie z.B. 1-Hydroxybenzotriazol (HOBt) (W. König, R. Geiger, Chem. Ber. 103, 788 (1970)) oder 3-Hydroxy-4-oxo-3,4-dihydrobenzotriazin (HOObt) (W. König, R. Geiger, Chem. Ber. 103, 2034 (1970)) zum Harz durchgeführt werden oder aber die Voraktivierung des Bausteines als aktivierter Ester kann separat erfolgen und die Lösung der aktivierten Spezies in einem geeigneten Lösungsmittel zum kupplungsfähigen Polymer gegeben werden.

Als Schutzgruppe für die exocyclische Aminofunktion der geschützten Nucleobase B' werden mit der gegen schwache Säuren labilen Aminoschutzgruppe PG kompatible Schutzgruppen verwendet. Bevorzugt werden Schutzgruppen, wie die Benzoyl-, Isobutanoyl-, Acetyl-, Phenoxyacetyl-, 4-(t-Butyl)-benzoyl-, 4-(t-Butyl)-Phenoxyacetyl-, 4-(Methoxy)-benzoyl-, 2-(4-Nitrophenyl)ethyl-oxycarbonyl-, 2-(2,4-Dinitrophenyl)ethyl-oxycarbonyl-, 9-Fluorenylmethoxycarbonyl-, Diphenylcarbamoyl- oder Formamidin-Gruppe verwendet. Insbesondere bevorzugt sind die Benzoyl-, Isobutanoyl-, 4-(t-Butyl)-benzoyl-, 2-(4-Nitrophenyl)ethyl-oxycarbonyl-, 2-(2,4-Dinitrophenyl)ethyl-oxycarbonyl-, 9-Fluorenylmethoxycarbonyl-, 4-(Methoxy)-benzoyl- oder para- (t-Butyl)-Phenoxyacetyl-, para-Nitrophenyl-2-ethyl-oxycarbonyl-Gruppe und für Guanin eine Kombination der 2-N-Acetyl mit der 6-O-Diphenylcarbamoyl-Gruppe.

Abspaltungsreagenzien für die gegen schwache Säuren labile Aminoschutzgruppe PG sind beispielsweise eine Lösung von 1-10% Trifluoressigsäure in Dichlormethan, eine Lösung von 1-10% Trichloressigsäure in Dichlormethan, eine Lösung von 2-15% Dichloressigsäure in Dichlormethan oder 1-5% p-Toluolsulfonsäure in Dichlormethan. Weiterhin kommen auch Lewis-Säuren, wie beispielsweise Bortrifluorid-etherat oder Zinkbromid in Dichlormethan/lsopropanol in Frage.

Die Aufkupplung der Aminosäurereste Q' bzw. A' der Formel la erfolgt mit Aminosäurederivaten, die vorzugsweise die gleiche Aminoschutzgruppe PG tragen, die auch für die Verbindungen der Formel IV verwendet wird. Eventuell vorhandene Seitenkettenfunktionen der Aminosäuren sind mit gegen Basen oder Alkalilauge labilen Schutzgruppen, wie z.B. 9-Fluorenylmethyl (Fm) oder 9-Fluorenymethoxycarbonyl (Fmoc) versehen. Bevorzugt sind hierbei Aminosäurederivate wie PG-Gly-OH, PG-Tic-OH, PG-Pro-OH, PG-Phe-OH, PG-Oic-OH, PG-Lys(Fmoc)-OH, PG-Arg(Fmoc)-OH, PG-Cys(Fm)-OH, PG-Asp(OFm)-OH, PG-Glu(OFm)-OH, PG-Aeg(Fmoc)-OH, PG-His(Trt)-OH, worin PG die obige Bedeutung hat. Ganz besonders bevorzugt sind hier die folgenden Aminosäurederivate: Mmt-Gly-OH, Mmt-Tic-OH, Mmt-Pro-OH, Mmt-Phe-OH, Mmt-Oic-OH, Mmt-Lys(Fmoc)-OH, Mmt-Arg(Fmoc)-OH, Mmt-Cys(Fm)-OH, Mmt-Asp(OFm)-OH, Mmt-Glu(OFm)-OH, Mmt-Aeg(Fmoc)-OH, Mmt-His(Fmoc)-OH.

Die Herstellung der bei dem oben beschriebenen Syntheseverfahren eingesetzten Verbindungen der Formel IV 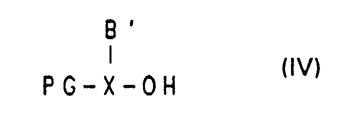 speziell in der Bedeutung 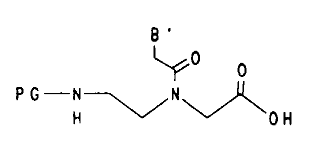 ist in der gleichzeitig eingereichten Patentanmeldung mit dem Titel "PNA-Synthese unter Verwendung einer basenlabilen Amino-Schutzgruppe" (HOE 94/F 059; DE-P 44 08 535.8) beschrieben.

Die oben beschriebenen PNA's werden durch Festphasensynthese an einem geeigneten Trägermaterial (z.B. Polystyrol, mit Polyoxyethylen modifiziertem Polystyrol, wie z.B. ®Tentagel, ®Controlled Pore Glass), das mit einer Ankergruppe L versehen ist, die latent den Rest Q^{o} enthält, aufgebaut. Die Festphasensynthese beginnt am C-terminalen Ende des PNA mit der Kupplung eines mit einer säurelabilen Schutzgruppe geschützten Monomers oder Aminosäure, die gegebenenfalls in der Seitenkettenfunktion geschützt ist, an ein entsprechendes Harz.

Nach Abspaltung der Schutzgruppe des an das Harz gekuppelten Bausteines mit einem geeigneten Reagenz, wie oben beschrieben, werden die nachfolgenden geschützten Bausteine (PNA-Monomere und Aminosäurederivate) nacheinander in der gewünschten Reihenfolge aufgekuppelt. Die intermediär entstehenden, mit einer säurelabilen Schutzgruppe am N-Terminus geschützten PNA-Harze werden vor der Verknüpfung mit dem nachfolgenden PNA-Monomeren durch die vorbeschriebenen Reagenzien entblockiert.

Die Kupplung bzw. Aktivierung der Aminosäurederivate mit einem der oben genannten Aktivierungsreagenzien kann in Dimethylformamid, N-Methylpyrrolidinon, Acetonitril oder Methylenchlorid oder einer Mischung aus den genannten Lösungsmitteln durchgeführt werden. Die vorgenannten Lösungsmittel können auch zusätzlich mit Hilfsbasen wie z.B. Pyridin, N-Ethylmorpholin oder Triethylamin versetzt werden. Das aktivierte Derivat wird üblicherweise in einem 1,5 bis 10 fachen Überschuß eingesetzt. In Fällen, in denen eine unvollständige Kupplung eintritt, wird die Kupplungsreaktion wiederholt, ohne Entblockierung der Aminogruppe des gerade aufgekuppelten Bausteins durchzuführen.

Verfahren zur Einführung des Restes R⁰ sind zum Beispiel für den Fall, daß dieser Rest eine Carbonsäurefunktion enthält, die oben für die Aufkupplung der Aminosäuren und PNA-Monomeren beschriebenen Methoden. Weitere Verfahren sind die Umsetzung von Isocyanaten wie z.B. Phenylisocyanat, Isothiocyanaten wie z.B. Fluoresceinisothiocyanat, Chlorameisensäurederivaten, wie z.B. Chlorformylcarbazol, Kohlensäureaktivestern wie z.B. Cholesterin-(4-nitrophenyl)carbonat, Acridinium-succinimidylcarbonat, Sulfochloriden wie z.B. Dansylchlorid.

Gegebenenfalls können in einem weiteren Schritt der Amino- und Carboxy-Terminus der Verbindungen der Formel I auch miteinander verbunden werden. Diese Verknüpfung erfolgt bevorzugt über eine Amidbindung zwischen den Seitenkettenfunktionen der Aminosäurereste A bzw. Q in der Bedeutung Lys, Glu, Asp, Aeg bzw. durch Herstellung einer Disulfidbrücke zwischen jeweils einer Aminosäure A und Q in der Bedeutung Cys.

Der vorhergehend beschriebene Syntheseablauf kann auch mittels kommerziell erhältlicher Syntheseautomaten, wie z.B. Peptidsynthesizern, Multiplen Peptidsynthesizern, DNA-Synthesizern unter leichter Modifikation der üblicherweise verwendeten Syntheseprogrammen durchgeführt werden.

Nach Synthese der PNA's in der vorgehend beschriebenen Weise kann das PNA-Oligomer vom Harz mit geigneten Reagenzien, wie z.B. konzentrierter Ammoniaklösung, Ethylendiamin, Hydrazin, Butylamin, Methylamin oder Ethanolamin abgespalten werden. Je nach Art des verwendeten Linkers und der Art der verwendeten Schutzgruppen werden Oligomer und die weiteren Seitenkettenschutzgruppen der Nucleinbasen simultan abgespalten. Das Abspaltungsreagenz kann dabei auch durch geeignete Lösungsmittel, wie z.B. Acetonitril, Ethanol oder Methanol, verdünnt angewendet werden.

Die Aufreinigung des nach der Abspaltung erhaltenen Roh-Oligomeres erfolgt mit in der Peptid- oder Nucleotidchemie üblichen Verfahren, wie z.B. HPLC, lonenaustauschchromatographie etc.

Die für Aminosäuren verwendeten Abkürzungen entsprechen dem in der Peptidchemie üblichen drei Buchstaben-Code wie er in Europ. J. Biochem. 138, 9 (1984) beschrieben ist. Weitere verwendete Abkürzungen sind nachfolgend aufgelistet.
- Aeg: N-(2-Aminoethyl)glycyl, -NH-CH₂-CH₂-NH-CH₂-CO-
- Aeg(A^{MeOBz}): N-(2-Aminoethyl)-N-((9-(N⁶-4-methoxybenzoyl)-adenosyl)acetyl)-glycyl
- Aeg(C^{Bz}): N-(2-Aminoethyl)-N-((1-(N⁴-benzoyl)-cytosyl)acetyl)-glycyl
- Aeg(C^{MeOBz}): N-(2-Aminoethyl)-N-((1-(N⁴-4-methoxybenzoyl)-cytosyl)acetyl)-glycyl
- Aeg(C^{tBuBz}): N-(2-Aminoethyl)-N-((1-(N⁴-4-tert.butylbenzoyl)-cytosyl)acetyl)-glycyl
- Aeg(G^{iBu}): N-(2-Aminoethyl)-N-((9-(N²-isobutanoyl)-guanosyl)acetyl)-glycyl
- Aeg(G^{2-Ac,4-Dpc}): N-(2-Aminoethyl)-N-((9-(N²-acetyl-O⁴-diphenylcarbamoyl)guanosyl)glycyl
- Aeg(T): N-(2-Aminoethyl)-N-((1-thyminyl)acetyl)-glycyl
- Bnpeoc: 2,2-[Bis(4-nitrophenyl)]-ethoxycarbonyl)
- Boc: tert.-Butyloxycarbonyl
- BOI: 2-(Benzotriazol-1-yl)oxy-1,3-dimethyl-imidazolidiniumhexafluorphosphat
- BOP: Benzotriazolyl-1-oxy-tris(dimethylamino)-phosphoniumhexafluorphosphat
- BroP: Brom-tris(dimethylamino)-phosphonium-hexafluorphosphat
- BSA: N,O-Bis-(trimethylsilyl)-acetamid
- But: tert.-Butyl
- Bz: Benzoyl
- Bzl: Benzyl
- CI-Z: 4-Chlor-benzyloxycarbonyl
- CPG: Controlled Pore Glas
- DBU: 1,8-Diazabicyclo[5.4.0]undec-7-en
- DCM: Dichlormethan
- Ddz: 3.5-Dimethoxyphenyl-2-propyl-2-oxycarbonyl
- DMF: Dimethylformamid
- Dmt: Di-(4-methoxyphenyl)phenylmethyl,
- Dnpeoc: 2-(2,4-Dinitrophenyl)-ethoxycarbonyl
- Dpc: Diphenylcarbamoyl
- FAM: Fluorescein-Rest
- Fm: 9-Fluorenylmethyl
- Fmoc: 9-Fluorenylmethyloxycarbonyl
- H-Aeg-OH: N-(2-Aminoethyl)glycin
- HAPyU: O-(7-Azabenzotriazol-1-yl)-1,1,3,3-bis(tetramethylen) uronium-hexafluorphosphat
- HATU: O-(7-Azabenzotriazol-1-yl)-1,1,3,3-tetramethyluroniumhexafluorphosphat
- HBTU: O-(Benzotriazol-1-yl)-1,1,3,3-tetramethyluronium-hexafluorphosphat
- HOBt: 1 -Hydroxybenzotriazol
- HONSu: N-Hydroxysuccinimid
- HOObt: 3-Hydroxy-4-oxo-3,4- dihydrobenzotriazin
- iBu: Isobutanoyl
- MeOBz: 4-Methoxybenzoyl
- Mmt: 4-Methoxytriphenylmethyl
- Moz: 4-Methoxybenzyloxycarbonyl
- MSNT: 2,4,6-Mesitylensulfonyl-3-nitro-1,2,4-triazolid
- Mtt: 4-Methylphenyl)diphenylmethyl
- NBA: Nitrobenzylalkohol
- NMP: N-Methylpyrrolidin
- Pixyl: 9-(9-Phenyl)xanthenyl
- PyBOP: Benzotriazolyl-1-oxy-tripyrrolidinophosphonium-hexafluorphosphat
- PyBroP: Brom-tripyrrolidinophosphonium-hexafluorphosphat
- TAPipU: O-(7-Azabenzotriazol-1 -yl)-1,1,3,3-bis(pentamethylen) uronium-tetrafluoroborat
- TBTU: O-(Benzotriazol-1-yl)-1,1,3,3-tetramethyluronium-tetrafluoroborat
- tBu: tert.-Butyl
- tBuBz: 4-tert. Butylbenzoyl
- TDBTU: O-(3,4-Dihydro-4-oxo-1,2,3-benzotriazin-3-yl)-1,1,3,3-tetramethyluronium-tetrafluoroborat
- TDO: 2,5-Diphenyl-2,3-dihydro-3-oxo-4-hydroxythiophendioxid
- TFA: Trifluoressigsäure
- THF: Tetrahydrofuran
- TNTU: O-[(5-Norbornen-2,3-dicarboximido]-1,1,3,3-tetramethyluronium-tetrafluoroborat
- TOTU: O-[(Cyano(ethoxycarbonyl)methylen)amino]-1,1,3,3-tetramethyluronium-tetrafluoroborat
- TPTU: O-(1,2-Dihydro-2-oxo-1-pyridyl)-1,1,3,3'-tetramethyluronium-tetrafluoroborat
- Trt: Trityl
- TSTU: O-(N-Succinimidyl)-1,1,3,3-tetramethyluronium-tetrafluoroborat
- Z: Benzyloxycarbonyl
- MS(ES⁺): Elektrospray Massenspektrum (Positiv lon)
- MS(ES⁻): Elektrospray Massenspektrum (Negativ lon)
- MS(DCI): Desorption Chemical lonisation Massenspektrum
- MS(FAB): East-Atom-Bombardment-Massenspektrum

Die folgenden Beispiele sollen die bevorzugten Methoden zur Herstellung der erfindungsgemäßen Verbindungen verdeutlichen, ohne daß die Erfindung darauf eingeschränkt wäre.

### Synthese der Peptide Nucleic Acids

Die Synthese der PNA's erfolgt beispielsweise an einem Ecosyn D-300 DNA Synthesizer (Fa. Eppendorf/Biotronik, Maintal) oder einem ABI 380B DNA Synthesizer (Fa. Applied Biosystems, Weitersstadt). Eine Beschreibung der Synthese-Zyklen ist nachfolgend ausgeführt.

Die Synthese erfolgt in Standard-DNA-Synthese-Säulen der Firma Applied Biosystems, die mit Mmt-hex-succ-Tentagel oder Mmt-hex-succ-CPG befüllt sind. Es werden Säulen für Synthesen im 3 µMol oder 6 µMol Maßstab verwendet. Als Reagenz zur Abspaltung der Mmt-Schutzgruppe wird 3% Trichloressigsäure in Dichlormethan verwendet. Nach Waschen des Trägers mit Acetonitril erfolgt eine Neutralisation mit einer 3.5 M Lösung von 4-Ethylmorpholin in Acetonitril. Zur Kupplung wird eine Mischung bestehend aus einer 0.3 bzw. 0.4 M Lösung der Mmt-Aeg-Derivate in Acetonitril/DMF, DMF/NMP mit 1 % Triton X-100, DMF mit N-Ethylmorpholin, DMF mit Pyridin, einer 0.9 M Lösung von PyBOP in Acetonitril und einer 3.5 M Lösung von 4-Ethylmorpholin in Acetonitril oder einer 0.3 M Lösung von HATU in DMF mit einer 0.3 M Lösung von NEM in DMF in die Synthesesäule eingebracht. Nachfolgendes Capping erfolgt mit einer 1:1 Mischung der Standard DNA-Synthese Capping-Reagenzien (Acetanhydrid/Lutidin/N-Methylimidazol-Lösung in THF). Das PNA wird durch Behandlung mit konzentrierter Ammoniak-Lösung am Synthesizer vom Träger abgespalten, wobei zur Entfernung der Basen-Schutzgruppen die vereinigten ammoniakalischen Lösungen in einer verschlossenen Ampulle für 5h auf 55°C erwärmt werden. Danach erfolgt gegebenenfalls die Abspaltung der aminoterminalen Mmt-Gruppe mit 80% Essigsäure bei Raumtemperatur.

Die PNA's werden an einem Beckman System Gold HPLC Gerät über eine Dionex Nucleopac PA-100 (4x250mm)-Säule mit einem linearen Gradienten von 0-0.75M NaCI in 20mM NaOH analysiert.

Die Reinigung erfolgt an einer Pharmacia Biopilot FPLC-Anlage mit einer Pharmacia Mono Q HR 10/10-Säule mit einem linearen Gradienten von 0-0.5M NaCI in 20mM NaOH als Elutionsmittel. Die Entsalzung der gereinigten PNA's gelingt mit Hilfe einer BondElut-C18-Säule (Fa. Analytichem Int'l) oder über ®Biogel (Fa. Biorad).

### Beispiel 1

### 1-Hydroxy-6-((4-methoxyphenyl)-diphenylmethylamino)-hexan

### (Mmt-hex)

6-Aminohexan-1-ol (lg; 8.55mmol) wird in wasserfreiem Pyridin (7ml) gelöst und mit Triethylamin (0.2ml) versetzt. Zu dieser Lösung gibt man innerhalb von 45 Minuten eine Lösung von (4-Methoxyphenyl)-diphenylmethyl chlorid (2.5g; 8.12mmol) in wasserfreiem Pyridin (9ml). Die Reaktionslösung wird 30 Minuten bei 22°C weitergerührt und durch Zugabe von Methanol (3ml) gestoppt. Die Lösung wird am Rotationsverdampfer eingeengt, der erhaltene Rückstand zur Entfernung des Pyridins dreimal mit Toluol koevaporiert. Der erhaltene Rückstand wird in Ethylacetat gelöst und diese Lösung nacheinander mit einer gesättigten Natriumbicarbonatlösung, mit Wasser und einer gesättigten Kaliumchloridlösung gewaschen. Nachdem die organische Phase über Na₂SO₄ getrocknet wurde, filtriert man und konzentriert die Lösung im Vakuum. Das Rohprodukt läßt sich durch Kieselgelchromatographie mit
Heptan: Ethylacetat:Triethylamin/ 49.5:49.5:1 reinigen.
Ausbeute: 1.64g
MS (FAB,NBA/LiCl) 396.3 (M+Li)⁺, 390.3 (M+H)⁺, 273.2 (MMT)⁺
R_{f} 0.44 (Heptan:Ethylacetat = 1:1),

### Beispiel 2

### 6-((4-Methoxyphenyl)-diphenylmethylamino)-hex-1-yl hemisuccinat

### (Mmt-hex-succ)

1-Hydroxy-6-((4-methoxyphenyl)-diphenylmethylamino)-hexan (1.00g; 2.57mmol) werden in wasserfreiem Pyridin (10ml) gelöst. Zu dieser Lösung gibt man Bernsteinsäureanhydrid (0.257g; 2.57mmol) and 4-Dimethylaminopyridin (31.3mg; 0.257mmol). Nach 3 Stunden Rühren bei 22°C gibt man weiteres Bernsteinsäureanhydrid (25.7mg; 0.257mmol) and 4,4-Dimethylaminopyridin (62.6mg; 0.56mmol) zu und erwärmt diese Lösung 6 Stunden lang auf 50°C. Nach weiteren 16 Stunden bei 22°C wird eingeengt, der Rückstand in Ethylaceteat aufgenommen und die erhaltene Lösung mit eiskalter 5%-iger wässriger Zitronensäure gewaschen. Nach Trocknen der org. Phase (Na₂SO₄) wird die Lösung am Rotationsverdampfer eingeengt. Die Reinigung des Rückstands durch Kieselgelchromatographie mit 50% CH₂Cl₂/1% Triethylamin in Ethylacetat und dann mit 5% Methanol/1% Triethylamin in Dichloromethan ergibt die gewünschte Verbindung in Form eines farblosen Öls.
MS (ES⁻) 978.0 (2M-H)⁻, 488.3 (M-H)⁻
R_{f} 0.30 (CH₂Cl₂:EthylAcetat = 1:1).

### Beispiel 3

### 6-((4-Methoxyphenyl)-diphenylmethylamino)-hex-1-yl succinylamido-Tentagel

### (Mmt-hex-succ-Tentagel)

Die Aminoform von Tentagel^{R} (Rapp Polymere)(0.5g; 0.11 mmol Aminogruppen) läßt man 10 Minuten in 4-Ethylmorpholin (0.1 ml) und DMF (5ml) quellen. Dann gibt man eine Lösung von 6-((4-Methoxyphenyl)-diphenylmethylamino)-hex-1-yl hemisuccinat (97.4mg; 0.165mmol), 4-Ethylmorpholine (15.9mg; 0.138mmol; 17.4ml) and TBTU (52.9mg; 0.165mmol) in DMF (3ml) zu und schüttelt die Suspension 16 Stunden lang bei 22°C. Der derivatisierte Tentagelträger wird abfiltriert und nacheinander mit DMF (3x3ml), CH₂Cl₂ (3x1ml) and Diethylether (3x1ml) gewaschen und getrocknet. Nicht reagierte Aminofunktionen werden durch 1-stündige Behandlung mit Acetanhydrid/Lutidin/1-Methylimidazol in THF (1ml) blockiert. Der fertige Träger wird mit CH₂Cl₂ (3x1ml) und Diethylether (3x1ml) gewaschen und im Vakuum getrocknet. Die Beladung bezogen auf die eingeführte Monomethoxytritylfunktion beträgt 168 µmolg⁻¹.

### Beispiel 4

### 6-((4-Methoxyphenyl)-diphenylmethylamino)-hex-1-yl succinylamidopropyl-Controlled-pore glass.

### (Mmt-hex-succ-CPG)

Die Herstellung erfolgt analog wie in Beispiel 3 beschrieben ausgehend von Aminopropyl-CPG (Firma Fluka) (550Å; 1.0g;) und 6-((4-Methoxyphenyl)-diphenylmethylamino)-hex-1-yl hemisuccinat (48.7mg; 0.082mmol), 4-Ethylmorpholine (7.6ml) und TBTU (26.4mg; 0.082mmol) in DMF (3ml). Die Beladung des MMT-hex-succCPG beträgt 91 µmolg⁻¹.

### Beispiel 5

### H-[Aeg(T)]₃-(hex)

H-[Aeg(T)]₃-(hex) wird nach oben beschriebenen Syntheseverfahren im 3 µMol Maßstab an Mmt-Hex-Succ-Tentagel synthetisiert. Als Monomer wird Mmt-Aeg(T)-OH verwendet. Die Rohausbeute beträgt 71 OD₂₆₀. Das von 2 OD aufgenommene Massenspektrum zeigt das gewünschte Produkt bei m/e 915.8 (M + H)⁺, und als Nebenprodukt H-[Aeg(T)]₂-(hex) bei m/e 650.5.

### Beispiel 6

### H-[Aeg(T)]-[Aeg(C)]-[Aeg(T)]-[Aeg(C)]-[Aeg(T)]₂-(hex)

H-[Aeg(T)]-[Aeg(C)]-[Aeg(T)]-[Aeg(C)]-[Aeg(T)]₂-(hex) wird nach oben beschriebenen Syntheseverfahren im 3 µMol Maßstab an Mmt-Hex-Succ-Tentagel synthetisiert. Als Monomere verwendet man Mmt-Aeg(T)-OH und Mmt-Aeg(C^{Bz})-OH. Die Rohausbeute beträgt 98.6 OD₂₆₀. 35 OD₂₆₀ des Rohproduktes werden gereinigt und entsalzt und ergaben 14.5 OD₂₆₀ der gewünschten Verbindung. Massenspektrometrische Analyse des gereinigten Produktes zeigt das gewünschte Produkt m/e 1685.0 (M + H)⁺ als einzigen Hauptpeak.

### Beispiel 7

### H-[Aeg(T)]-[Aeg(C)]-[Aeg(T)]-[Aeg(C)]-[Aeg(T)]₂-(hex)

H-[Aeg(T)]-[Aeg(C)]-[Aeg(T)]-[Aeg(C)]-[Aeg(T)]₂-(hex) wird nach oben beschriebenen Syntheseverfahren im 3 µMol Maßstab an Mmt-Hex-Succ-Tentagel synthetisiert. Als Monomere verwendet man Mmt-Aeg(T)-OH und Mmt-Aeg(C^{tBuBz})-OH.

### Beispiel 8

### H-[Aeg(A)]-[Aeg(C)]-[Aeg(A)]-[Aeg(T)]-[Aeg(C)]-[Aeg(A)]-[Aeg(T)]-[Aeg(G)]-[Aeg(G)]-[Aeg(T)]-[Aeg(C)]-[Aeg(G)] (hex)

Die Synthese der PNA's erfolgt mit einem Ecosyn D-300 DNA Synthesizer (Fa. Eppendorf/Biotronik, Maintal) an 130 mg (5 µmol) Mmt-Hex-Succ- Aminoproyl-CPG.

Für die Synthese wurden folgende Lösungen eingesetzt:

| | | |
|---|---|---|
| 1) | Aktivator-Lsg.: | 0,3molare HATU-Lösung in getrocknetem DMF |
| 2) | Base für Aktivierung: | 0,3molare Lösung von NEM in getrocknetem DMF |
| 3) | Abspaltung von Mmt | 3%ige Lösung von Trichloressigsäure in Dichlormethan |
| 4) | Neutralisations-Lsg. | Tetrahydrofuran/Wasser/Pyridin 7:2:1 |
| 5) | Mmt-Aeg(T)-OH: | 0,3molare Lösung in 0,3molarer Lösung von NEM in getrocknetem DMF |
| 6) | Mmt-Aeg(A^{MeOBz})-OH: | 0,3molare Lösung in 0,3molarer Lösung von NEM in getrocknetem DMF |
| 7) | Mmt-Aeg(C^{MeOBz})-OH: | 0,3molare Lösung in 0,3molarer Lösung von NEM in getrocknetem DMF |
| 8) | Mmt-Aeg(G^{iBu})-OH: | 0,3molare Lösung in 0,3molarer Lösung von NEM in getrocknetem DMF |

Nach Beendigung der Synthese wird der PNA-CPG-Träger getrocknet und wie oben beschrieben aufgearbeitet.
Ausbeute: 245 OD₂₆₀,
MS 3369.6(ES⁺): (M)⁺

## Patentansprüche

1. Verfahren zur Herstellung von PNA-Oligomeren der Formel I 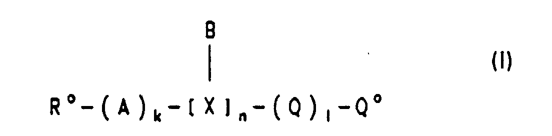 worin
B/X für 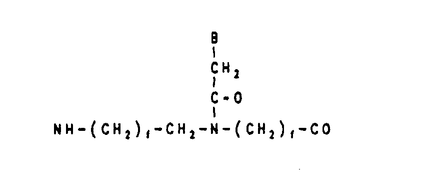 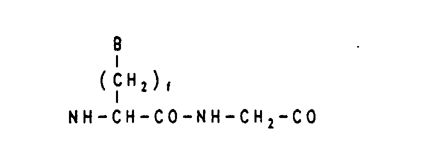 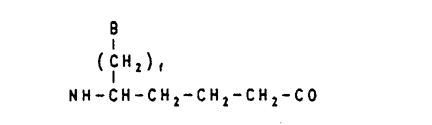 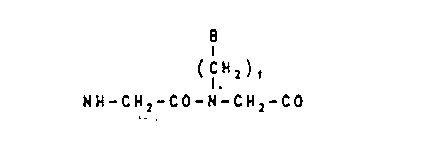 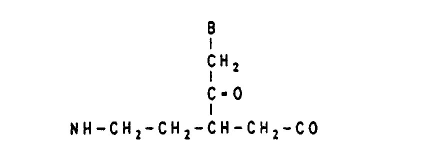 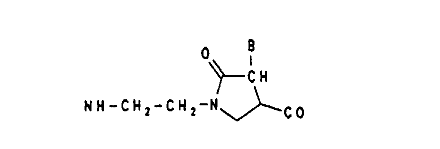 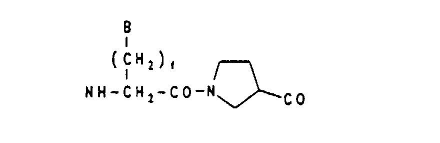 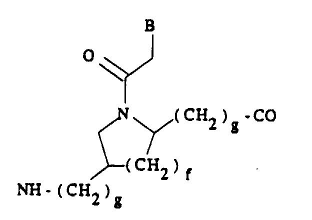 mit f = 1 - 4 und g = Null - 3 steht;
R⁰ Wasserstoff, C₁-C₁₈-Alkanoyl, C₁-C₁₈-Alkoxycarbonyl, C₃-C₈-Cycloalkanoyl, C₇-C₁₅-Aroyl, C₃-C₁₃-Heteroaroyl, oder eine Gruppe bedeutet, die die intrazelluläre Aufnahme des Oligomers begünstigt oder die bei der Hybridisierung mit der target-Nucleinsäure interagiert;
A ein Aminosäurerest bedeutet;
k eine ganze Zahl von Null bis 10 ist;
Q ein Aminosäurerest bedeutet;
I eine ganze Zahl von Null bis 10 ist;
B für eine in der Nucleotidchemie übliche natürliche Nucleobase oder nichtnatürliche Nucleobase oder deren Prodrugformen, oder auch für Basenersatzverbindungen steht;
Q^{o} Hydroxy, NH₂ oder NHR" bedeutet mit R" = C₁-C₁₈-Alkyl, C₂-C₁₈-Aminoalkyl oder C₂-C₁₈-Hydroxyalkyl; und
n eine ganze Zahl von 1 - 50 ist,
dadurch gekennzeichnet, daß man
a) auf einen polymeren Träger der Formel II
L-[Polymer] (II),
der mit einer Ankergruppe L versehen ist, die latent den Rest Q° enthält entweder zuerst mit einem für die Festphasensynthese üblichen Verfahren Aminosäuren (Q') aufkuppelt,
b) die gegen schwache Säuren labile Schutzgruppe PG mittels eines geeigneten Reagenzes abspaltet,
c) die Schritte a und b (I-1)fach wiederholt,
d) auf die dabei als Zwischenprodukt entstehende Verbindung der Formel III
(Q')_{I}-L-[Polymer] (III),
worin L wie oben definiert ist, Q' für eine Aminosäure Q steht, bei der die gegebenenfalls vorhandenen Seitenkettenfunktionen geschützt sind und I für eine ganze Zahl von Null bis 10 steht oder direkt auf den polymeren Träger der Formel II eine Verbindung der Formel IV 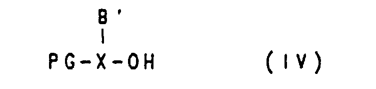 worin
PG für eine gegen schwache Säuren labile Aminoschutzgruppe und B'/X für einen Baustein wie in Formel I definiert steht,
mit einer an der exocyclischen Amino -bzw. Hydroxyfunktion gegebenenfalls geschützten Nucleobase, worin
B' für in der Nucleotidchemie übliche natürliche Nucleobasen oder nichtnatürliche Nucleobasen oder deren Prodrugformen, oder auch für Basen ersatzverbindungen, deren exocyclische Amino bzw. Hydroxygruppen gegebenenfalls durch geeignete bekannte Schutzgruppen geschützt sind, stehen,
unter Verwendung der in der Peptidchemie üblichen Kupplungsreagenzien aufkuppelt,
e) die temporäre gegen schwache Säuren labile Schutzgruppe PG mittels eines geeigneten Reagenzes abspaltet,
f) die Schritte d und e (n-1)fach wiederholt,
g) mit einem für die Festphasensynthese üblichen Verfahren weitere Aminosäuren (A') aufkuppelt,
h) die gegen schwache Säuren labile Schutzgruppe PG mittels eines geeigneten Reagenzes abspaltet,
i) die Schritte g und h (k-1)fach wiederholt,
j) für den Fall, daß R⁰ nicht Wasserstoff ist, den Rest R⁰ mit einem üblichen Verfahren einführt,
k) aus der als Zwischenverbindung erhaltenen Verbindung der Formel la 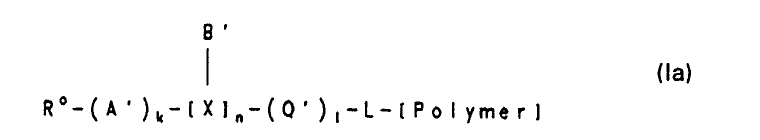 worin R⁰, k, B'/X, n, Q', und I wie oben definiert sind, A' für eine Aminosäure A steht, die gegebenenfalls in der Seitenkette geschützt ist, und L für eine Ankergruppe steht,
die Verbindung der Formel I mittels eines Abspaltungsreagenzes vom polymeren Träger abspaltet wobei gleichzeitig oder auch anschließend die an der exocyclischen Amino- bzw. Hydroxyfunktion der Nucleobasen und an den Seitenketten der Aminosäuren gegebenenfalls vorhandenen Schutzgruppen abgespalten werden.

2. Verfahren zur Herstellung von PNA-Oligomeren der Formel I gemäß Anspruch 1, dadurch gekennzeichnet, daß
A ein Aminosäurerest aus der Reihe Glycin, Leucin, Histidin, Phenylalanin, Cystein, Lysin, Arginin, Asparaginsäure, Glutaminsäure, Prolin, Tetrahydroisochinolin-3-carbonsäure, Octahydroindol-2-carbonsäure und N-(2-Aminoethyl)glycin bedeutet;
k eine ganze Zahl von Null bis 6 ist;
Q ein Aminosäurerest aus der Reihe Glycin, Leucin, Histidin, Phenylalanin, Cystein, Lysin, Arginin, Asparaginsäure, Glutaminsäure, Prolin, Tetrahydroisochinolin-3-carbonsäure, Octahydroindol-2-carbonsäure und N-(2-Aminoethyl)glycin bedeutet;
I eine ganze Zahl von Null bis 6 ist;
B für eine natürliche Nucleobase ausgewählt aus Adenin, Cytosin, Guanin, Thymin und Uracil oder eine nichtnatürliche Nucleobase ausgewählt aus Purin, 2.6-Diaminopurin, 7-Deazaadenin, 7-Deazaguanin, N⁴N⁴-Ethanocytosin, N⁶N⁶-Ethano-2.6-diaminopurin, 5-Methylcytosin, 5-(C₃-C₆)-Alkinyl-uracil, 5-(C₃-C₆)-Alkinyl-uracil, cytosin, 5-Fluor-uracil oder Pseudoisocytosin, 2-Hydroxy-5-methyl-4-triazolopyrimidin oder deren Prodrugformen, oder für Imidazol, Nitro-lmidazol oder Triazol steht; und
n eine ganze Zahl von 4 - 35 ist.

## Claims

1. A process for the preparation of PNA oligomers of the formula I 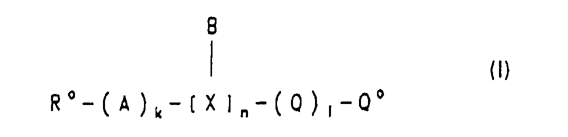 in which
B/X is 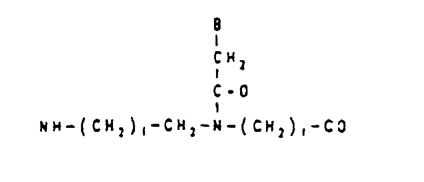 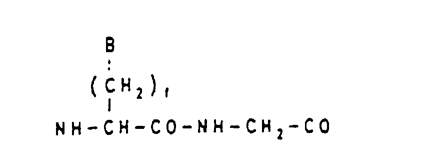 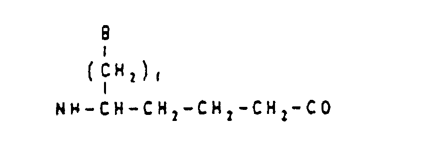 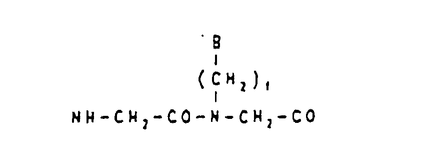 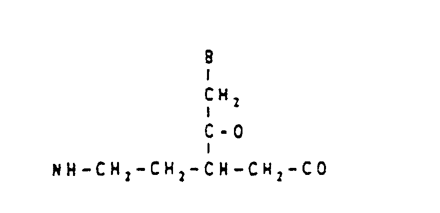 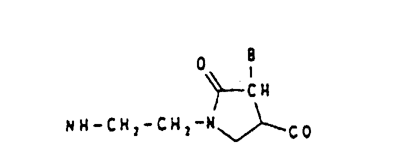 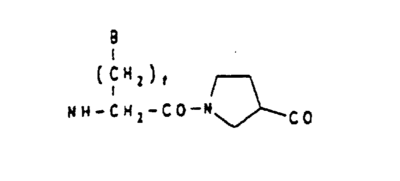 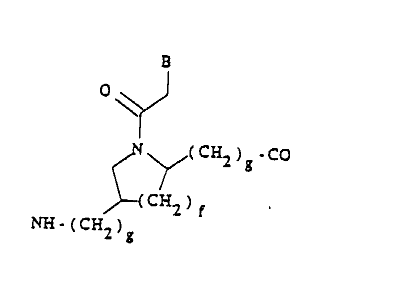 where f is 1 - 4 and g is zero - 3;
R⁰ is hydrogen, C₁-C₁₈-alkanoyl, C₁-C₁₈-alkoxycarbonyl, C₃-C₈-cycloalkanoyl, C₇-C₁₅-aroyl, C₃-C₁₃-heteroaroyl, or a group which favors intracellular uptake of the oligomer or which interacts with the target nucleic acid during the hybridization;
A is an amino acid radical;
k is an integer from zero to 10;
Q is an amino acid radical;
l is an integer from zero to 10;
B is a natural nucleotide base or non-natural nucleotide base conventionally used in nucleotide chemistry or their prodrug forms, or else base substitute compounds;
Q^{o} is hydroxyl, NH₂ or NHR", in which R" is C₁-C₁₈-alkyl, C₂-C₁₈-aminoalkyl or C₂-C₁₈-hydroxyalkyl; and
n is an integer from 1 - 50,
which comprises
a) either firstly coupling amino acids (Q¹) onto a polymeric support of the formula II
L-[polymer] (II),
which is provided with an anchoring group L which contains the radical Q^{o} in latent form, using a process conventionally used in solid-phase synthesis,
b) cleaving off the protective group PG which is labile to weak acids, using a suitable reagent,
c) repeating steps a and b (l-1) times,
d) coupling onto the compound of the formula III, which is formed as an intermediate
(Q')₁-L-[polymer] (III),
in which L is as defined above, Q' is an amino acid Q in which the side chain functions which are optionally present are protected, and 1 is an integer from zero to 10, a compound of the formula IV 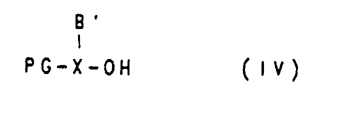 in which
PG is an amino protective group which is labile to weak acids and
B'/X is a unit as defined in formula I,
provided with a nucleotide base which is optionally protected on the exocyclic amino or hydroxyl function in which
B' are natural nucleotide bases or non-natural nucleotide bases conventionally used in nucleotide chemistry or their prodrug forms or else base substitute compounds, whose exocyclic amino or hydroxyl groups are optionally protected by suitable known protective groups,
or else coupling a compound of the formula IV directly onto the polymeric support of the formula II,
using the coupling reagents conventionally used in peptide chemistry,
e) cleaving off the temporary protective group PG which is labile to weak acids by means of a suitable reagent,
f) repeating steps d and e (n-1) times,
g) coupling on further amino acids (A') using a process conventionally used in solid-phase synthesis,
h) cleaving off the protective group PG which is labile to weak acids by means of a suitable reagent,
i) repeating steps g and h (k-1) times,
j) in the event that R⁰ is not hydrogen, introducing the radical R⁰ using a customary process, and
k) cleaving off the compound of the formula I from the polymeric support out of the compound of the formula Ia obtained as intermediate 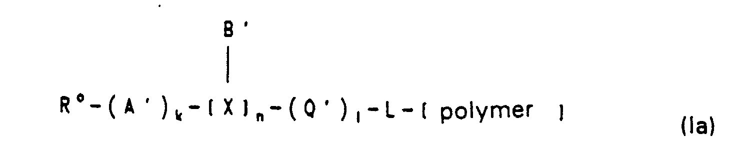 in which R⁰, k, B'/X, n, Q' and l are as defined above, A' is an amino acid A which is optionally protected in the side chain and L is an anchoring group,
using a cleaving reagent, during which process the protective groups which are optionally present on the exocyclic amino or hydroxyl function of the nucleotide bases and on the side chains of the amino acids are simultaneously or else subsequently cleaved off.

2. A process for the preparation of PNA oligomers of the formula I as claimed in claim 1, wherein
A is an amino acid radical from the series consisting of glycine, leucine, histidine, phenylalanine, cysteine, lysine, arginine, aspartic acid, glutamic acid, proline, tetrahydroisoquinoline-3-carboxylic acid, octahydroindole-2-carboxylic acid and N-(2-aminoethyl) glycine;
k is an integer from zero to 6;
Q is an amino acid radical from the series consisting of glycine, leucine, histidine, phenylalanine, cysteine, lysine, arginine, aspartic acid, glutamic acid, proline, tetrahydroisoquinoline-3-carboxylic acid, octahydroindole-2-carboxylic acid and N-(2-aminoethyl)glycine;
l is an integer from zero to 6;
B is a natural nucleotide base selected from adenine, cytosine, guanine, thymine and uracil, or a non-natural nucleotide base selected from purine, 2,6-diaminopurine, 7-deazaadenine, 7-deazaguanine, N⁴N⁴-ethanocytosine, N⁶N⁶-ethano-2,6-diaminopurine, 5-methylcytosine, 5-(C₃-C₆)alkynyl-uracil, 5-(C₃-C₆)alkynylcytosine, 5-fluorouracil or pseudoisocytosine, 2-hydroxy-5-methyl-4-triazolopyrimidine, or their prodrug forms, or is imidazole, nitroimidazole or triazole; and
n is an integer from 4 - 35.

## Revendications

1. Procédé pour la préparation d'oligomères de PNA (acides nucléiques peptidiques) de formule I 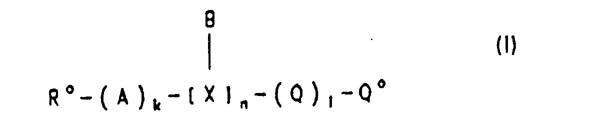 dans laquelle
B/X représentent 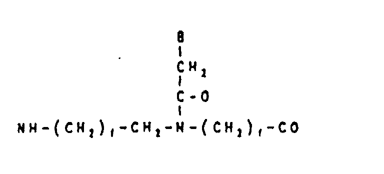 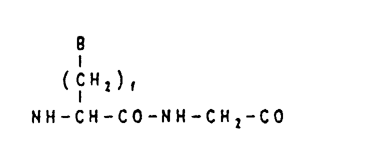 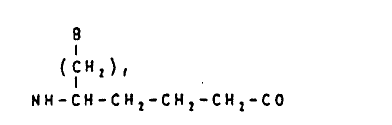 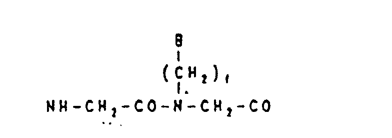 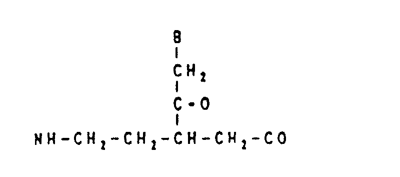 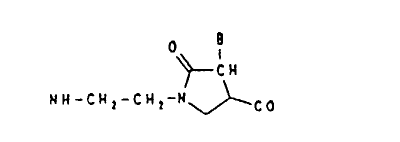 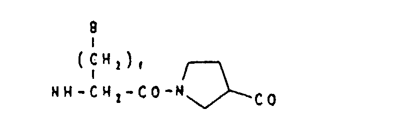 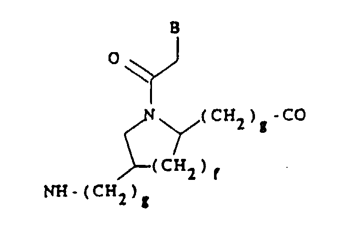 où f = 1 - 4 et g = zéro - 3;
R^{o} représente un atome d'hydrogène ou un groupe alcanoyle en C₁-C₁₈, alcoxy(C₁-C₁₈)carbonyle, cycloalcanoyle en C₃-C₈, aroyle en C₇-C₁₅, hétéroaroyle en C₃-C₁₃ ou un groupe qui facilite la capture intracellulaire de l'oligomère ou qui entre en interaction lors de l'hybridation avec l'acide nucléique cible;
A représente un reste d'aminoacide;
k est un nombre entier allant de zéro à 10;
Q représente un reste d'aminoacide;
l est un nombre entier allant de zéro à 10;
B représente une base nucléique naturelle ou une base nucléique non naturelle, usuelles dans la chimie des nucléotides, ou leurs précurseurs, ou bien des composés de remplacement de bases;
Q^{o} représente un groupe hydroxyle, NH₂ ou NHR", R" étant un groupe alkyle en C₁-C₁₈, aminoalkyle en C₂-C₁₈ ou hydroxyalkyle en C₂-C₁₈; et
n est un nombre entier allant de 1 à 50,
caractérisé en ce que
a) sur un support polymère de formule II
L-[polymère] (II)
qui est muni d'un groupe d'ancrage L qui contient de façon latente le radical Q^{o}, on attache d'abord des aminoacides (Q') à l'aide d'un procédé usuel pour la synthèse en phase solide,
b) on élimine au moyen d'un réactif approprié le groupe protecteur PG labile en présence d'acides faibles,
c) on répète (l-1) fois les étapes a et b,
d) sur le composé de formule III
(Q')ₗ-L-[polymère] (III),
dans laquelle L est tel que défini ci-dessus, Q' représente un aminoacide Q dans lequel les fonctions de chaîne latérale éventuellement présentes sont protégées et 1 représente un nombre entier allant de zéro à 10, ainsi formé en tant que produit intermédiaire, ou directement sur le support polymère de formule II, on attache, avec utilisation des réactifs de couplage usuels dans la chimie des peptides, un composé de formule IV 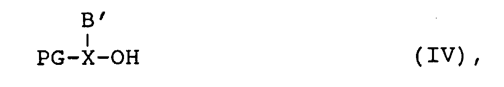 dans laquelle
PG représente un groupe protecteur de fonction amino labile en présence d'acides faibles et
B' /X représente un composant tel que défini dans la formule I,
avec une base nucléique éventuellement protégée sur la fonction amino ou hydroxyle exocyclique, où
B' représente des bases nucléiques naturelles ou des bases nucléiques non naturelles, usuelles dans la chimie des nucléotides, ou leurs précurseurs, ou bien des composés de remplacement de bases, dont les groupes hydroxyle ou amino exocyliques sont éventuellement protégés par des groupes protecteurs connus appropriés,
e) on élimine à l'aide d'un réactif approprié le groupe protecteur PG temporaire, labile en présence d'acides faibles,
f) on répète (n-1) fois les étapes d) et e),
g) on attache d'autres aminoacides (A') à l'aide d'un procédé usuel pour la synthèse en phase solide,
h) on élimine à l'aide d'un réactif approprié le groupe protecteur PG labile en présence d'acides faibles,
i) on répète (k-1) fois les étapes g) et h),
j) dans le cas où R^{o} n'est pas un atome d'hydrogène, on introduit le reste R^{o} à l'aide d'un procédé usuel,
k) à partir du composé de formule Ia 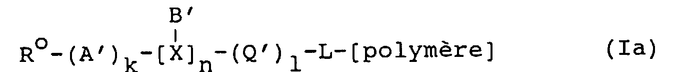 dans laquelle R^{o}, k, B'/X, n, Q' et 1 sont tels que définis plus haut, A' représente un aminoacide A qui est éventuellement protégé sur la chaîne latérale et L représente un groupe d'ancrage, obtenu en tant que composé intermédiaire,
on sépare le composé de formule I du support polymère au moyen d'un réactif de séparation en éliminant en même temps ou bien ensuite les groupes protecteurs éventuellement présents sur la fonction amino ou hydroxyle exocyclique des bases nucléiques et sur les chaînes latérales des aminoacides.

2. Procédé pour la préparation d'oligomères de PNA de formule I selon la revendication 1, caractérisé en ce que
A représente un reste d'aminoacide choisi parmi la glycine, la leucine, l'histidine, la phénylalanine, la cystéine, la lysine, l'arginine, l'acide aspartique, l'acide glutamique, la proline, l'acide tétrahydro-isoquinoléine-3-carboxylique, l'acide octahydro-indole-2-carboxylique et la N-(2-aminoéthyl)glycine;
k est un nombre entier allant de zéro à 6;
Q représente un reste d'aminoacide choisi parmi la glycine, la leucine, l'histidine, la phénylalanine, la cystéine, la lysine, l'arginine, l'acide aspartique, l'acide glutamique, la proline, l'acide tétrahydro-isoquinoléine-3-carboxylique, l'acide octahydro-indole-2-carboxylique et la N-(2-aminoéthyl)glycine;
l est un nombre entier allant de zéro à 6;
B représente une base nucléique naturelle choisie parmi l'adénine, la cytosine, la guanine, la thymine et l'uracile ou une base nucléique non naturelle choisie parmi la purine, la 2,6-diaminopurine, la 7-déazaguanine, la N⁴N⁴-éthanocytosine, la N⁶N⁶-éthano-2,6-diaminopurine, la 5-méthylcytosine, un 5-alcynyl(C₃-C₆)-uracile, une 5-alcynyl(C₃-C₆)-cytosine, le 5-fluoro-uracile ou la pseudo-isocytosine, la 2-hydroxy-5-méthyl-4-triazolo-pyrimidine ou leurs précurseurs, ou représente l'imidazole, le nitro-imidazole ou le triazole; et
n est un nombre entier allant de 4 à 35.
